# EUROPÄISCHE PATENTSCHRIFT

(11) **EP 0 799 012 B1**
(45) Veröffentlichungstag und Bekanntmachung des Hinweises auf die Patenterteilung: **08.03.2000**
(21) Anmeldenummer: 95942731.1
(22) Anmeldetag: 22.12.1995
(51) Int. Cl.: A61J 3/10, B65B 9/04, B30B 11/16, A61K 9/20

(54) **VERFAHREN ZUR HERSTELLUNG VON UMHÜLLTEN TABLETTEN**
METHOD OF PRODUCING COATED TABLETS
PROCEDE DE FABRICATION DE COMPRIMES ENROBES

(30) Priorität: 23.12.1994 DE 4446468
(43) Veröffentlichungstag der Anmeldung: 08.10.1997
(73) Patentinhaber: BASF AKTIENGESELLSCHAFT, 67056 Ludwigshafen (DE)
(72) Erfinder: ROSENBERG, Joerg, D-67158 Ellerstadt (DE); MAIER, Werner, D-67105 Schifferstadt (DE); GRABOWSKI, Sven, D-67061 Ludwigshafen (DE); BREITENBACH, Jörg, D-68199 Mannheim (DE)
(74) Vertreter: Kinzebach, Werner, Dr.
(86) Internationale Anmeldenummer: EP9505118
(87) Internationale Veröffentlichungsnummer: WO9619963

(56) Entgegenhaltungen:
- EP-A- 0 227 060
- DE-A- 1 766 546
- FR-A- 2 531 907
- US-A- 2 513 852
- US-A- 4 880 585

## Beschreibung

Die vorliegende Erfindung betrifft ein Verfahren zur Herstellung von umhüllten Tabletten durch Formen einer wirkstoffhaltigen Schmelze in einem Kalander mit gegenläufig rotierenden Formwalzen, die an ihrer Oberfläche Vertiefungen zur Aufnahme und Formung der Tablettenmasse aufweisen (Schmelzkalandrierung).

Die Herstellung von Tabletten durch Kalandrierung einer wirkstoffhaltigen Schmelze ist aus der DE-A- 1 766 546 und der US-A-4,880,585 bekannt. Grundlage dieses Verfahren ist die Einbettung eines Wirkstoffes in eine Schmelze aus einem Träger, z.B. Fettsubstanzen oder wasserlösliche, thermoplastische Polymere. Die Schmelze wird dadurch erzeugt, daß die Mischung aus Wirkstoff, Polymer und gegebenenfalls weiteren Hilfsstoffen beispielsweise in einem Extruder aufgeschmolzen und als Schmelze in einem nachgeschalteten Formkalander zu Tabletten geformt wird, die durch Abkühlen aushärten. Der Formkalander umfaßt ein sich gegenläufig drehendes Formwalzenpaar, wobei die Formwalzen auf ihrer Oberfläche Gravuren (Vertiefungen) aufweisen, die der Form einer Hälfte der gewünschten Tablette entsprechen. Die Tablettenformung erfolgt im Berührungsbereich der beiden Walzen durch Kombination der Tablettenmasse einer Vertiefung auf der einen Walze mit derjenigen der gegenüberliegenden Vertiefung auf der anderen Walze.

Der größte Teil der auf dem Markt befindlichen Tabletten wird als sog. Filmtabletten hergestellt, d.h. auf die Tabletten wird im letzten Produktionsschritt eine dünne Schicht aus wasserlöslichen Polymeren aufgebracht. Dieses "Filmcoating" ist aus verschiedenen Gründen oft unverzichtbar, da z.B.
a) ein von den verwendeten Wirk- und/oder Hilfsstoffen angebrachter Geschmack überdeckt werden muß, bis sich die Tablette im Magen befindet,
b) der verwendete Wirkstoff instabil z.B. gegenüber Licht Feuchtigkeit etc. ist,
c) die Tabletten zur leichteren Identifizierung einen farbigen Überzug benötigen.

Die aufgetragenen Schutzschichten ("das Coating") werden bisher nahezu ausschließlich durch Aufsprühen von Lösungen aus wasserlöslichen Polymeren (organische Lösungsmittel und/oder Wasser) unter gleichzeitigen Trocknen durchgeführt. Neben dem heute üblichen Filmcoating (Schichtdicke im Mikrometerbereich) existiert das Dragierverfahren, bei dem dicke, teilweise im Millimeterbereich liegende Schichten aus zuckerhaltigen Mischungen aufgebracht werden. Diese breit angewandten Technologien werden in verschiedenen Lehrbüchern beschrieben (s.H. Sucker, P. Fuchs, P. Speiser: Pharmazeutische Technologie; 2. Auflage, G. Thieme Verlag Stuttgart (1991), Seiten 347-368).

Falls ein Überzug über die durch Schmelzkalandrierung hergestellten Tabletten gewünscht war, mußte dieser Überzug nach dem Abkühlen der Tabletten in einem eigenen Arbeitsgang aufgebracht werden. Dies erfolgte in konventioneller Weise, beispielsweise durch Aufsprühen in rotierenden Trommeln, nach dem Tauchrohrverfahren oder in der Wirbelschicht etc.

Die konventionellen Verfahren zur Aufbringung von Coatingschichten bzw. zum Dragieren verlangen alle einen vergleichsweise extrem hohen Energieeinsatz, da die verwendeten Lösungsmittel aus den Sprühlösungen nach dem Aufsprühen auf die Tabletten schnell wieder entfernt werden müssen. Zudem dauert ein Coating-Prozeß meist mehrere Stunden, da die Sprührate nicht beliebig hoch eingestellt werden kann.

Das Aufbringen des Überzugs in einem eigenen Arbeitsgang erfordert somit einen erheblichen Zeitaufwand, zusätzliche Maschinen und zusätzliches Personal, was sich deutlich auf die Herstellungskosten auswirkt.

Der vorliegenden Erfindung liegt daher die Aufgabe zugrunde, ein Verfahren zur Herstellung von umhüllten Tabletten durch Schmelzkalandrierung zur Verfügung zu stellen, bei dem eine Umhüllung der Tabletten auf einfache, kostensparende Weise möglich ist.

Überraschenderweise wurde nun gefunden, daß diese Aufgabe gelöst wird, wenn man Tabletten durch Schmelzkalandrierung herstellt und dabei die wirkstoffhaltige Schmelze zwischen zwei Folien aus dem Umhüllungsmaterial in die Kalanderformwalzen einführt.

Gegenstand der vorliegenden Erfindung ist daher ein Verfahren zur Herstellung von umhüllten Tabletten durch Formen einer wirkstoffhaltigen Schmelze in einem Kalander mit zwei gegenläufig rotierenden Formwalzen, die an ihrer Oberfläche einander gegenüberliegende Vertiefungen zur Aufnahme und Formung der Tablettenmasse aufweisen, das dadurch gekennzeichnet ist, daß man die wirkstoffhaltige Schmelze zwischen zwei Folien aus dem Umhüllungsmaterial in die Formwalzen einführt.

Die Herstellung der Tabletten erfolgt ausgehend von einer Mischung, die einen oder mehrere pharmazeutische Wirkstoffe sowie einen oder mehrere übliche Hilfsstoffe enthält und die durch Schmelzen oder Erweichen mindestens einer Komponente teigig bis zähflüssig (thermoplastisch) und daher extrudierbar wird.

Die pharmazeutische Mischung wird dann in üblicher Weise aufgeschmolzen, vorzugsweise in einem Extruder, und dem Formkalander zugeführt, wie das beispielsweise in der US-A-4,880,585 beschrieben ist.

Gleichzeitig mit der Schmelze werden den sich entlang einer Mantellinie berührenden oder in nur sehr geringem Abstand voneinander befindlichen Formwalzen zwei Folien, die das Umhüllungsmaterial bilden, so zugeführt, daß die Folien jeweils zwischen Formwalze und Schmelze zu liegen kommen. Im weiteren Verlauf der Kalandrierung kommt es zur Formung der Tablettenmasse zu der gewünschten Tablettenform, wobei gleichzeitig das die Umhüllung der Tabletten bildende Teil aus der Folie ausgestanzt und auf die Tabletten aufgebracht wird. Durch die an den Formwalzen herrschenden Temperaturen, die im allgemeinen bei 50 bis 150°C liegen, kommt es zu einem Erweichen des Folienmaterials und damit zu einer Umhüllung der Tabletten. Das Folienmaterial verschmilzt an den Rändern der Tabletten und hüllt dabei die Tabletten einzeln ein, so daß die Tablette vollständig und gleichmäßig mit dem Umhüllungsmaterial überzogen ist.

Falls erforderlich, werden die umhüllten Tabletten anschließend einem Kühlvorgang unterzogen, beispielsweise in einem Luft- oder Kühlbad.

Das erfindungsgemäße Verfahren hat den Vorteil, daß die bei der konventionellen Tablettenherstellung diskontinuierlich ablaufenden Einzelprozesse Granulieren, Tablettieren und Coaten in einem einzigen Verfahrensschritt zusammengefaßt sind, der zudem kontinuierlich erfolgt. Darüber hinaus erfordert das Aufbringen des Überzugs (Coating) keinen zusätzlichen Energieaufwand, da es gleichzeitig mit der Tablettierung (hier: Kalandrierung) erfolgt, die ohnehin bei höheren Temperaturen durchgeführt wird.

Gemäß einer bevorzugten Ausführungsform verwendet man Folien, die zur Ausbildung eines Filmüberzugs auf den Tabletten geeignet sind, so daß man Filmtabletten erhält. Die Schichtdicke des Films ist über einen weiten Bereich variierbar. Dies ist beim konventionellen Aufsprühen nur über Veränderung der Prozeßzeiten möglich (kürzere/längere Sprühdauer). Insbesondere bei dickeren Schichten zeigt sich die Überlegenheit des neuen Verfahrens (Einsparung von Zeit), da diese dicken Schichten extrem schnell und sehr gleichmäßig aufgebracht werden können. Im allgemeinen verwendet man Folien, die eine Dicke von etwa 10 µm bis 500 µm aufweisen. Es ist möglich, Folien unterschiedlicher Dicke einzusetzen, so daß die Tablettenober- und die Tablettenunterhälfte mit einem Film unterschiedlicher Dicke überzogen ist, wodurch z.B. die Auflösungscharakteristik der Tablette im Magen-Darmtrakt gezielt beeinflußt werden kann.

Das Folienmaterial kann aus einer breiten Palette von Materialien ausgewählt werden. Voraussetzung ist lediglich, daß es sich um ein pharmazeutisch akzeptables Material handelt. Das Folienmaterial kann so gewählt werden, daß man eine Tablette erhält, die sich bereits im Magensaft auflöst oder daß man eine Tablette mit modifizierter Wirkstofffreisetzung erhält, beispielsweise eine Tablette mit magensaftresistentem Überzug oder eine Tablette mit Langzeitwirkung, beispielsweise eine Tablette vom Sustained-Release-Typ, Prolonged-Release-Typ, Repeat-Release-Typ oder Delayed-Release-Typ.

Für die Herstellung derartiger Filmtabletten geeignete Folienmaterialien, die sich rasch im sauren Magensaft auflösen, sind insbesondere Gelatine, Polyvinylalkohol, Alkylcellulosen, wie Methylcellulosen, Hydroxyalkylcellulosen, wie Hydroxyethyl-, Hydroxypropyl- oder Hydroxypropylmethylcellulose, Polyvinylpyrrolidon, bestimmte Acrylharze, wie Copolymerisate auf Basis von Dimethylaminoethylmethacrylat und Methacrylestern (Eudragit E) etc. allein oder in Mischungen untereinander.

Beispiele für erfindungsgemäß brauchbare Filmbildner für Überzüge mit modifizierter Wirkstofffreisetzung sind Alkylcellulosen, wie Ethylcellulose, Polyvinylester, wie Polyvinylacetat, bestimmte Acrylharze, wie Copolymerisate auf Basis von Methacrylsäure und Methacrylsäureester (Eudragit L und S), Cellulosephthalate, wie Celluloseacetatphthalat oder Hydroxypropylmethylcellulosephthalat, etc. Die Freisetzungscharakteristik läßt sich außerdem dadurch beeinflussen, daß man Folien unterschiedlichen Materials verwendet, wobei für die Umhüllung einer oder beider Tablettenhälften auch mehrere Folien zur Anwendung kommen können.

Bei Verwendung wasserlöslicher Folien haben sich Polymere mit thermoplastischer Verarbeitbarkeit wie z.B. Hydroxyalkylcellulosen, Gelatine oder Acrylharze als Folienmaterial besonders bewährt. Sie können in einer Dicke von etwa 50 bis 150 µm zur Anwendung kommen und bilden dabei einen dünnen, sehr gleichmäßigen wasserlöslichen Überzug auf den Tabletten.

Das erfindungsgemäße Verfahren ermöglicht eine weitgehend aseptische Herstellung der Tabletten. Durch das Aufschmelzen der Tablettenmasse und, wenn dies in einem Extruder erfolgt, durch den intensiven Scherenergie-Eintrag in das Produkt werden die Keime in der Masse abgetötet, so daß diese als steriles Produkt den Formwalzen zugeführt wird. Wenn man dann sterilisierte Polymerfolien einsetzt und die Schmelzkalandrierung unter aseptischen Bedingungen durchführt, beispielsweise mit keimfreier Luft (Laminar-Flow), erhält man die Tabletten in steriler Form. Die Tabletten können dann in einem weiteren Verfahren steril verpackt werden oder, was besonders bevorzugt ist, simultan mit der Formung der Tabletten verblistert werden (siehe die nachfolgenden Ausführungen). In letzterem Falle ist die Gefahr der Produktkontamination mit pathogenen Keimen im Vergleich zu einem konventionellen Verfahren mit separater Verpackung erheblich reduziert.

Erfindungsgemäß können die Folien auch einen weiteren Wirkstoff enthalten. Dabei kann es sich um einen Wirkstoff handeln, der mit einer der Komponenten in der Tablettenmasse nicht kompatibel ist. Die nicht-kompatiblen Bestandteile werden auf diese Weise voneinander getrennt gehalten. Durch die Aufnahme eines Wirkstoffes in die Folie ist es aber auch möglich, durch den in der Folie enthaltenen Wirkstoff eine Initialdosis freizusetzen und mit der eigentlichen Tablette dann für eine weitere Einzeldosis oder für die Erhaltung der Arzneimittelkonzentration zu sorgen.

Gemäß einer weiteren Ausführungsform verwendet man als Folien solche, die für eine Verpackung der Tabletten geeignet sind. Es handelt sich dabei insbesondere um wasserunlösliche Tiefziehfolien, wobei als Material Polyethylen, Polypropylen, Polyvinylchlorid, Polyethylenterephthalat, Polystyrol, Aluminium oder beschichtetes Aluminium bevorzugt ist. Auf diese Weise werden die Tabletten sofort in eine Blisterpackung eingesiegelt. Der sonst übliche eigene Verpackungsschritt entfällt somit und außerdem ist es auf diese Weise möglich, wie oben erwähnt, die Tabletten auf einfachste Weise aseptisch zu verpacken, insbesondere dann, wenn man dafür Sorge trägt, daß die Außenkanten des Tablettenbandes luftdicht verschweißt werden.

Überraschenderweise hat sich gezeigt, daß nicht, wie erwartet, eine intensive Verklebung der heißen Tablettenmasse mit der wasserunlöslichen Tiefziehfolie erfolgt, so daß die spätere Entnahme der Tabletten aus der Verpackung behindert oder sogar unmöglich gewesen wäre.

Für die Verpackung der Tabletten hat es sich als besonders vorteilhaft erwiesen, eine Formwalze mit den Vertiefungen für die Aufnahme und Formung der Tablettenmasse mit einer Glattwalze zu kombinieren. Man erhält auf diese Weise "Halb"-Tabletten, die in eine Blisterpackung eingesiegelt sind, welche auf einer Seite Vertiefungen für die Aufnahme der Tabletten aufweist und auf der anderen Seite mit einer glatten, abziehbaren Folie verschlossen ist. In diesem Fall hat sich eine Aluminiumfolie oder eine Folie aus beschichtetem Aluminium für den Verschluß der Verpackung als besonders zweckmäßig erwiesen.

Es kann sich als zweckmäßig erweisen, die verpackten Tabletten nicht, wie sonst üblich, an der Luft abkühlen zu lassen sondern einen eigenen Abkühlungsschritt vorzusehen. Hierfür ist ein Wasserbad, kalter Luftstrom etc. geeignet. Auf diese Weise wird vermieden, daß die Tabletten in der Verpackung zu langsam erkalten, was zu nachträglichen Verformungen der Tabletten führen kann.

Es ist auch möglich, die Folien für den Filmüberzug der Tabletten und die Folien für die Verblisterung der Tabletten gleichzeitig zu verwenden. In diesem Fall wird die Schmelze in den Formwalzen von der Folie für den Filmüberzug umhüllt und gleichzeitig in die Verpackungsfolie eingesiegelt. Damit ist es möglich, sämtliche zur Tablettenherstellung erforderlichen Grundoperationen, nämlich Tablettierung, Coating und Verpackung in einem einzigen Schritt durchzuführen, der zudem in kontinuierlicher Arbeitsweise erfolgt. Damit sind enorme Kosteneinsparungen verbunden.

In manchen Fällen hat es sich als zweckmäßig erwiesen, die Formwalzen oder die zur Anwendung kommenden Folien, insbesondere deren Außenseiten, mit einem Formentrennmittel zu überziehen, um das Ablösen der Tabletten bzw. der Verpackung von den Formwalzen zu erleichtern. Geeignete Formentrennmittel sind z.B. Siliconharze, Stearinsäure, Calcium- oder Magnesiumstearat, Paraffin, Cetylalkohol oder Lecithine.

Mit dem erfindungsgemäßen Verfahren ist es auch auf einfache Weise möglich, den Folien weitere Additive zuzusetzen. Derartige Additive sind beispielsweise Farbpigmente, wobei die Ober- und Unterseite der Tabletten bzw. der Verpackung unterschiedliche Farbe aufweisen kann, Geschmackkorrigenzien, Weichmacher etc. Auch können eine oder beide Folien bedruckt sein, z.B. mit Zahlen, Namen etc., um eine eindeutige Identifizierung der Tabletten durch die Patienten zu gewährleisten. Bisher konnte dies nur durch nachträgliches Bedrucken mit Tintenstrahldruckern erfolgen.

Die Form der Vertiefungen und damit der Tabletten kann weitgehend beliebig gewählt werden. Besonders zweckmäßig sind längliche und ellipsoidsegmentartige Vertiefungen, so daß man Oblong-Tabletten und linsenförmige Tabletten erhält.

Gewünschtenfalls können auch teilbare Tabletten hergestellt werden. Zu diesem Zweck kann man am Boden der Vertiefungen eine kleine oft im Mikrometerbereich liegende Rippe vorsehen, die zur Ausbildung der Bruchrille in den fertiggestellten Tabletten führt. Vorzugsweise aber verwendet man wenigstens eine Formwalze, bei der die Vertiefungen durch mindestens einen Steg unterteilt sind, der sich im wesentlichen bis zur Mantelfläche der Formwalze erstreckt und die Ausbildung der Bruchrille bewirkt.

Bei der oben erwähnten Mischung für die Herstellung der Tabletten handelt es sich insbesondere um Mischungen, die pharmakologisch akzeptable Polymere enthalten (wobei die Glastemperatur der Mischung unter der Zersetzungstemperatur aller Mischungskomponenten liegt), beispielsweise Polyvinylpyrrolidon (PVP), Copolymerisate von N-Vinylpyrrolidon (NVP) und Vinylestern, insbesondere Vinylacetat, Copolymerisate von Vinylacetat und Crotonsäure, teilverseiftes Polyvinylacetat, Polyvinylalkohol, Ethylen/Vinylacetat-Copolymerisate, Polyhydroxyethylmethacrylat, Copolymerisate von Methylmethacrylat und Acrylsäure, Celluloseester, Celluloseether, insbesondere Hydroxypropylcellulose, Polyethylenglykol oder Polyethylen, bevorzugt NVP-Copolymerisate mit Vinylacetat, Hydroxypropylcellulose und Polyethylenglycole/Polyethylenoxide. Die K-Werte (nach H. Fikentscher, Cellulose-Chemie 13 (1932), Seiten 58 bis 64 und 71 und 74) der Polymeren liegen im Bereich von 10 bis 100, vorzugsweise 12 bis 70, insbesondere 12 bis 35, für PVP vorzugsweise bei 12 bis 35, insbesondere bei 12 bis 17.

Das polymere Bindemittel muß in der Gesamtmischung aller Komponenten im Bereich von 50 bis 180, vorzugsweise 60 bis 130°C erweichen oder schmelzen, so daß die Masse extrudierbar ist. Die Glasübergangstemperatur der Mischung muß also auf jeden Fall unter 180, vorzugsweise unter 130°C liegen. Erforderlichenfalls wird sie durch übliche pharmakologisch akzeptable weichmachende Hilfsstoffe wie langkettige Alkohole, Ethylenglykol, Propylenglykol, Trimethylolpropan, Triethylenglykol, Butandiole, Pentanole, Hexanole, Polyethylenglykole, Silicone, aromatische Carbonsäureester (z.B. Dialkylphthalate, Trimellithsäureester, Benzoesäureester, Terephthalsäureester) oder aliphatische Dicarbonsäureester (z.B. Dialkyladipate, Sebacinsäureester, Azelainsäureester, Zitronen- und Weinsäureester) oder Fettsäureester herabgesetzt.

Übliche galenische Hilfsstoffe, deren Gesamtmenge bis zu 100 Gew.-% bezogen auf das Polymerisat, betragen kann, sind z.B. Streckmittel wie Silikate oder Kieselerde, Stearinsäure oder deren Salze, z.B. das Magnesium- oder Kalziumsalz, Methylcellulose, Natrium-Carboxymethylcellulose, Talkum, Saccharose, Lactose, Getreide- oder Maisstärke, Kartoffelmehl, Polyvinylalkohol, ferner Netz-, Konservierungs-, Spreng-, Adsorptionsmittel, Farbstoffe, Geschmacksstoffe (vgl. z.B. H. Sucker et al. Pharmazeutische Technologie, Thieme-Verlag, Stuttgart 19/8). Einzige Voraussetzung für deren Eignung ist eine ausreichende Temperaturstabilität.

Unter pharmazeutischen Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, daß sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.-% liegen. Auch Wirkstoff-Kombinationen können eingesetzt werden. Wirkstoffe im Sinne der Erfindung sind auch Vitamine und Mineralstoffe, sowie Pflanzenbehandlungsmittel und Insektizide.

Das erfindungsgemäße Verfahren ist beispielsweise zur Verarbeitung folgender Wirkstoffe geeignet:

Acebutolol, Acetylcystein, Acetylsalicylsäure, Acyclovir, Albrazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium Hydroxid, Benzocain, Benzoesäure, Betamethason, Bezafibrat, Biotin, Biperiden, Bisoprolol, Prazosin, Bromazepam, Bromhexin, Bromocriptin, Budesonid, Bufexamac, Buflomedil, Buspiron, Coffein, Campher, Captopril, Carbamazepin, Carbidopa, Carboplatin, Carotinoide wie beispielsweise β-Carotin oder Canthaxanthin, Cefachlor, Cefalexin, Cefatroxil, Cefazolin, Cefixim, Cefotaxim, Ceftazidim, Ceftriaxon, Cefuroxim, Celedilin, Chloramphenicol, Chlorhexidin, Chlorpheniramin, Chlortalidon, Cholin, Cyclosporin, Cilastatin, Cimetidin, Ciprofloxacin, Cisapride, Cisplatin, Clarithromycin, Clävulansäure, Clomibramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Diltiazem, Diphenhydramin, Dipyridamol, Dipyron, Disopyramid, Domperidon, Dopamin, Enalapril, Ephedrin, Epinephrin, Ergocalciferol, Ergotamin, Erythromycin, Estradiol, Ethinylestradiol, Etoposide, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gemfibrozil, Gentamicin, Ginkgo Biloba, Glibenclamid, Glipizid, Clozapin, Glycyrrhiza glabra, Guaifenesin, Haloperidol, Heparin, Hyaluronsäure, Hydrochlorothiazid, Hydrocodon, Hydrocortison, Hydromorphon, Ipratropium Hydroxid, Ibuprofen, Imipenem, Indomethacin, Iohexol, Iopamidol, Isosorbid-Dinitrat, Isosorbid-Mononitrat, Isotretinoin, Ketotifen, Ketoconazol, Ketoprofen, Ketorolac, Labatalon, Lactulose, Lecithin, Levocarnitin, Levodopa, Levoglutamide, Levonorgestrel, Levothyroxin, Lidocain, Lipase, Liponsäure, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrendipin, Nizatidin, Norethisteron, Norfloxacin, Norgestrel, Nortriptylin, Nystatin, Ofloxacin, Omeprazol, Ondanseron, Pancreatin, Panthenol, Pantothensäure, Paracetamol, Penicillin G, Penicillin V, Phenobarbital, Phenoxifyllin, Phenylephrin, Phenylpropanolamin, Phenytoin, Piroxicam, Polymyxin B, Povidone-Iod, Pravastatin, Prednisolon, Bromocriptin, Propafenon, Propranolol, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulpiridid, Tamoxifen, Tegafur, Teprenon, Terazosin, Terbutalin, Terfenadin, Theophyllin, Thiamin, Tiolopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin B₁, B₂, B₄, B₆, B₁₂, D₃, E, K, Volinsäure, Zidovudin.

Im einzelnen kann es zur Ausbildung von festen Lösungen kommen. Der Begriff "feste Lösungen" ist dem Fachmann geläufig, beispielsweise aus der eingangs zitierten Literatur. In festen Lösungen von pharmazeutischen Wirkstoffen in Polymeren liegt der Wirkstoff molekulardispers im Polymer vor.

Die nachfolgenden Beispiele erläutern die Erfindung, ohne sie zu beschränken.

### Beispiel 1

Eine Mischung, bestehend aus 60,0 Gew.-% Kollidon VA-64 (BASF) (Polyvinylpyrrolidon) - Copolymer mit Vinylacetat (60:40) und 40,0 Gew.-% Lactose-Monohydrat, wurde in einem Zweischnecken-Extruder (ZSK-40, Fa. Werner + Pfleiderer) unter folgenden Bedingungen extrudiert:
- Temperaturen:
   Schuß 1: 80°C
   Schuß 2: 100°C
   Schuß 3: 130°C
   Schuß 4: 130°C
   Düsen: 135°C
- Materialdurchsatz: 25 kg/h
- Schneckendrehzahl: 160 U/min

Die Schmelze 5 wurde zusammen mit zwei etwa 300 Mikrometer dicken Polypropylen-Folien 2 (Tiefzieh-Blisterfolie) in den Formkalander mit zwei Formwalzen 1, die sich in Pfeilrichtung drehen, gegeben (ca. 14 cm nutzbare Formwalzenbreite). Die Vertiefungen 3 der Formwalzen 1 waren so beschaffen, daß aus der Schmelze etwa 1000 mg schwere Oblong-Tabletten (20 x 8,5 mm) 4 geformt wurden. Das Material verließ den Kalander als in PP-Folie verpacktes Tablettenband. Die Tabletten waren nicht einzeln in der Folie eingeschweißt, da die Formwalzen des Kalanders so eingestellt waren, daß sie sich an keiner Stelle direkt berührten (ca. 0,1 mm Abstand). Die PP-Folie konnte nach Abkühlen leicht als etwa 14 cm breites Band (7 parallele Tablettenreihen auf der Formwalze) von den Tabletten entfernt werden. Eine Verklebung der Schmelze mit der PP-Folie fand in keinem Fall statt. Es war möglich, einzelne Tablettenreihen bzw. das Tablettenband in ganzer Formwalzenbreite durch nachgeschaltete Verschweißung der Außenkanten zu versiegeln (Luftabschluß).

Die im Rahmen der Erfindung verwendbaren Kalander- und Formwalzen können in an sich bekannter Weise gekühlt oder geheizt werden, und die für den jeweiligen Verarbeitungsprozeß optimale Oberflächentemperatur der Formwalze kann auf diese Weise eingestellt werden.

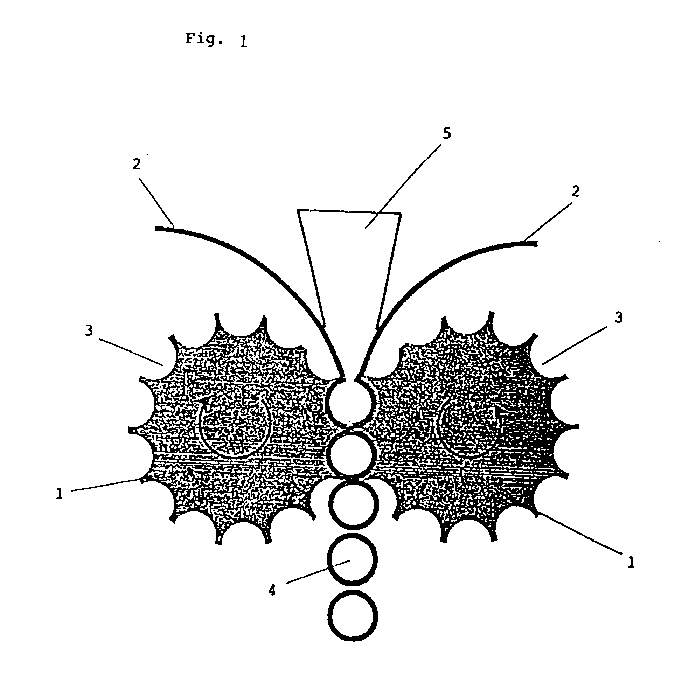

## Patentansprüche

1. Verfahren zur Herstellung von umhüllten Tabletten durch folgende Schritte:
(i) Bereitstellen einer Mischung, die einen oder mehrere pharmazeutische Wirkstoffe sowie einen oder mehrere übliche Hilfsstoffe erhält und die durch Schmelzen oder Erweichen mindestens einer Komponente teigig bis zähflüssig wird,
(ii) Aufschmelzen der pharmazeutischen Mischung und
(iii) Formen der wirkstoffhaltigen Schmelze (5) in einem Kalander mit gegenläufig rotierenden Formwalzen (1), die auf ihrer Oberfläche Vertiefungen (3) zur Aufnahme und Formung der Tablettenmasse aufweisen, wobei man die wirkstoffhaltige Schmelze zwischen zwei Folien (2) aus dem Umhüllungsmaterial in die Formwalzen einführt.

2. Verfahren nach Anspruch 1, dadurch gekennzeichnet, daß man Folien verwendet, die zur Ausbildung eines Filmüberzugs auf den Tabletten geeignet sind.

3. Verfahren nach Anspruch 2, dadurch gekennzeichnet, daß man Folien verwendet, die einen magensaftresistenten Überzug oder einen Überzug für eine modifizierte Wirkstofffreisetzung ergeben.

4. Verfahren nach Anspruch 2 oder 3, dadurch gekennzeichnet, daß das Folienmaterial ein Polymer umfaßt, das ausgewählt ist unter Gelatine, Polyvinylalkohol, Alkylcellulosen, Hydroxyalkylcellulosen, Celluloseestern, Carboxymethylcellulosen, Cellulosephthalaten, Polyvinylpyrrolidon, Polyvinylestern und Acrylharzen.

5. Verfahren nach einem der vorhergehenden Ansprüche, dadurch gekennzeichnet, daß die Folien zusätzlich den gleichen oder einen anderen Wirkstoff als die Tabletten enthalten.

6. Verfahren nach Anspruch 1, dadurch gekennzeichnet, daß man Folien verwendet, die geeignet sind, die Tabletten als Verpackung zu umhüllen.

7. Verfahren nach Anspruch 6, dadurch gekennzeichnet, daß man eine Polyethylen-, Polypropylen-, Polyvinylchlorid-, Polyethylenterephthalat-, Polystyrol-, Aluminium- oder beschichtete Aluminiumfolie verwendet.

8. Verfahren nach einem der vorhergehenden Ansprüche, dadurch gekennzeichnet, daß man die Folien für die Ausbildung eines Filmüberzugs gleichzeitig mit den Folien für die Verpackung einführt.

9. Verfahren nach einem der vorhergehenden Ansprüche, dadurch gekennzeichnet, daß man Folien aus unterschiedlichen Materialien und/oder Folien mit unterschiedlicher Dicke verwendet.

10. Verfahren nach einem der vorhergehenden Ansprüche, dadurch gekennzeichnet, daß die Folien Farbpigmente oder Geschmackskorrigenzien enthalten und/oder mit einem Trennmittel beschichtet sind.

11. Verfahren nach einem der vorhergehenden Ansprüche, dadurch gekennzeichnet, daß man zwei Formwalzen kombiniert, die unterschiedliche Vertiefungen aufweisen.

12. Verfahren nach einem der Ansprüche 1 bis 10, dadurch gekennzeichnet, daß man eine Formwalze mit Vertiefungen mit einer Glattwalze kombiniert.

13. Verfahren nach einem der vorhergehenden Ansprüche, dadurch gekennzeichnet, daß man zumindest eine Formwalze verwendet, bei der die Vertiefungen durch mindestens einen Steg unterteilt sind, der sich im wesentlichen bis zur Mantelfläche der Formwalze erstreckt und die Ausbildung einer Bruchrille bewirkt.

14. Verfahren nach einem der vorhergehenden Ansprüche, dadurch gekennzeichnet, daß die Formwalzen mit einem Formentrennmittel überzogen sind.

15. Verfahren nach einem der vorhergehenden Ansprüche, dadurch gekennzeichnet, daß man die umhüllten Tabletten zur Aushärtung kühlt.

## Claims

1. A process for the production of covered tablets by the following steps:
(i) preparing a mixture which contains one or more pharmaceutical active ingredients and one or more conventional ancillary substances and which becomes pasty to viscous by melting or softening of at least one component,
(ii) melting the pharmaceutical mixture and
(iii) molding the melt (5) containing active ingredient in a calender with counter-rotating molding rolls (1) which have on their surface depressions (3) for receiving and molding the tablet composition, with the melt containing active ingredient being introduced between two sheets (2) of the covering material into the molding rolls.

2. A process as claimed in claim 1, wherein sheets suitable for forming a film coating on the tablets are used.

3. A process as claimed in claim 2, wherein sheets which provide an enteric coating or a coating for modified release of active ingredient are used.

4. A process as claimed in claim 2 or 3, wherein the sheet material comprises a polymer which is selected from gelatin, polyvinyl alcohol, alkylcelluloses, hydroxyalkylcelluloses, cellulose esters, carboxymethylcelluloses, cellulose- phthalates, polyvinylpyrrolidone, polyvinyl esters and acrylic resins.

5. A process as claimed in any of the preceding claims, wherein the sheets additionally contain an active ingredient which is the same as or different from that in the tablets.

6. A process as claimed in claim 1, wherein sheets suitable for covering the tablets as packaging are used.

7. A process as claimed in claim 6, wherein a polyethylene, polypropylene, polyvinyl chloride, polyethylene terephthalate, polystyrene, aluminum or coated aluminum sheet is used.

8. A process as claimed in any of the preceding claims, wherein the sheets for forming a film coating are introduced at the same time as the sheets for the packaging.

9. A process as claimed in any of the preceding claims, wherein sheets of different materials and or sheets which differ in thickness are used.

10. A process as claimed in any of the preceding claims, wherein the sheets comprise colored pigments or masking flavors and/or are coated with a release agent.

11. A process as claimed in any of the preceding claims, wherein two molding rolls which have different depressions are combined.

12. A process as claimed in any of claims 1 to 10, wherein a molding roll with depressions is combined with a smooth roll.

13. A process as claimed in any of the preceding claims, wherein at least one molding roll in which the depressions are divided by at least one bar which extends essentially to the surface of the molding roll and forms a score is used.

14. A process as claimed in any of the preceding claims, wherein the molding rolls are coated with a mold release agent.

15. A process as claimed in any of the preceding claims, wherein the covered tablets are cooled for hardening.

## Revendications

1. Procédé pour la préparation de comprimés enrobés comprenant les étapes suivantes:
(i) fourniture d'un mélange, qui contient une ou plusieurs substances à activité pharmaceutique, ainsi qu'un ou plusieurs adjuvants classiques, et qui est rendu pâteux ou très visqueux par la fusion ou le ramollissement d'au moins un composant,
(ii) fusion du mélange pharmaceutique et
(iii) mise en forme de la masse fondue contenant les substances actives (5) dans une calandre ayant des rouleaux de calandrage tournant en sens opposé (1), qui présentent sur leur surface des creux (3) pour la prise et la mise en forme de la masse des comprimés, tandis que l'on introduit la masse fondue contenant les substances actives entre deux feuilles (2) de matière d'enrobage dans les rouleaux de calandrage.

2. Procédé selon la revendication 1, caractérisé par le fait qu'on utilise des feuilles qui conviennent pour la formation d'un revêtement pelliculaire sur les comprimés.

3. Procédé selon la revendication 2, caractérisé par le fait qu'on utilise des feuilles qui donnent un revêtement résistant au suc gastrique ou un revêtement pour une libération modifiée de substance active.

4. Procédé selon la revendication 2 ou 3, caractérisé par le fait que le matériau en feuille comprend un polymère qui est choisi parmi la gélatine, le poly(alcool vinylique), les alkylcelluloses, les hydroxyalkylcelluloses, les esters de cellulose, les carboxyméthylcelluloses, les phtalates de cellulose, la poly(pyrrolidone de vinyle), les poly(esters vinyliques) et les résines acryliques.

5. Procédé selon l'une des revendications précédentes, caractérisé par le fait que les. feuilles contiennent en outre la même ou une autre substance active que les comprimés.

6. Procédé selon la revendication 1, caractérisé par le fait qu'on emploie des feuilles qui sont appropriées pour envelopper les comprimés pour constituer un emballage.

7. Procédé selon la revendication 6, caractérisé par le fait qu'on emploie une feuille de polyéthylène, de polypropylène, de poly(chlorure de vinyle), de poly(téréphtalate d'éthylène), de polystyrène, d'aluminium ou d'aluminium revêtu.

8. Procédé selon l'une des revendications précédentes, caractérisé par le fait qu'on met en oeuvre les feuilles pour la formation d'un revêtement pelliculaire simultanément avec les feuilles pour l'emballage.

9. Procédé selon l'une des revendications précédentes, caractérisé par le fait qu'on emploie des feuilles de matériaux différents et/ou des feuilles d'épaisseurs différentes.

10. Procédé selon l'une des revendications précédentes, caractérisé par le fait que les feuilles contiennent des pigments colorés ou des agents pour corriger le goût et/ou sont revêtues avec un agent de démoulage.

11. Procédé selon l'une des revendications précédentes, caractérisé par le fait qu'on combine deux rouleaux de calandrage qui présentent des creux différents.

12. Procédé selon l'une des revendications 1 à 10, caractérisé par le fait qu'on combine un rouleau de calandrage ayant des creux avec un rouleau lisse.

13. Procédé selon l'une des revendications précédentes, caractérisé par le fait qu'on emploie au moins un rouleau de calandrage dans lequel les creux sont séparés par au moins une nervure, qui s'étend pratiquement jusqu'à la surface d'enveloppe du rouleau de calandrage et exerce la fonction de cannelure de rupture.

14. Procédé selon l'une des revendications précédentes, caractérisé par le fait que les rouleaux de calandrage sont revêtus avec un agent de démoulage.

15. Procédé selon l'une des revendications précédentes, caractérisé par le fait qu'on refroidit les comprimés enrobés jusqu'à leur durcissement.
